# EUROPEAN PATENT SPECIFICATION

(11) **EP 2 329 858 B1**
(45) Date of publication and mention of the grant of the patent: **10.07.2013**
(21) Application number: 09177728.4
(22) Date of filing: 02.12.2009
(51) Int. Cl.: A61M 16/06

(54) **Respiratory nasal mask with pivotable arm for holding and orientating the forehead support**
Nasale Atemmaske mit schwenkbarem Arm zum Halten und Ausrichten der Stirnstütze
Masque respiratoire nasal avec bras pivotant pour maintenir et orienter le support frontal

(43) Date of publication of application: 08.06.2011
(73) Proprietor: Air Liquide Medical Systems, 92182 Antony Cedex (FR)
(72) Inventor: Alberici, Luca, 25128 Brescia (IT); Rivetti, Alberto, 25038 Rovato (BS) (IT); Sandoni, Giuseppe, 25124 Brescia (IT)
(74) Representative: Pittis, Olivier

(56) References cited:
- WO-A1-00/78384
- WO-A2-03/082406
- WO-A2-2007/021777
- WO-A2-2008/036625
- US-A1- 2004 177 850
- US-A1- 2007 062 537
- US-A1- 2008 135 050

## Description

The invention concerns a respiratory mask with a pivotable forehead support, in particular a nasal mask, for use in the treatment of respiratory conditions or diseases, such as obstructive sleep apnea, that comprises a mechanism for pivoting the forehead support with respect to the mask body.

Nasal masks are commonly used for delivering non-invasive positive pressure ventilation (NPPV) or for nasal continuous positive airway pressure (N-CPAP) therapy in sleep disordered breathing (SDB) conditions, such as obstructive sleep apnea (OSA).

Nasal masks deliver a flow of breathable gas for, or to assist in, patient respiration, especially during the night, i.e., when the patient is sleeping.

Such a mask assembly typically comprises a rigid or semi-rigid hollow shell defining a breathing chamber that receives at least a part of the patient's nose and further comprising a soft face-contacting cushion that comes into contact with the patient's face, and a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face. An example of a nasal mask of this kind is given by EP-A-462701.

The shell typically receives a gas supply line which delivers a respiratory gas, such as air under pressure, into the breathing chamber of the shell. Further, the soft face-contacting cushion can be made of a soft, resilient elastomeric material that may conform to the various facial contours of the patient face.

The mask assembly is secured to the wearer's head by straps or similar devices thereby forming a headgear that can be adjusted to pull the mask against the face with sufficient force to achieve a gas tight seal between the mask and the wearer's face as taught by EP-A-462701, EP-A-874667, EP-A-1972357 or WO-A-00/57942.

However, with the current masks, it is often difficult to obtain an efficient positioning of the mask on the patient's face and to ensure sufficient tightness (seal) preventing the escape of gas as well as a comfort of use for the patient. In particular, as the morphology of the face is different from one user to another, it is important to be able to correctly position the forehead support of the mask on the user's forehead and afterwards maintain it in a desired position, whatever the forehead morphology of the patient is.

For instance, some masks including various mechanisms for positioning the forehead support are disclosed by US-A-2245658, EP-A-1985327 and EP-A-1356842.

Document WO 00/78384 discloses a breathing mask having features as in the preamble of the appended claim 1.

However, so far, while attempts for modifying the existing positioning mechanisms have been made, the resulting masks are not totally satisfying.

Hence, the problem to be solved is to provide an improved mask architecture allowing an easy and efficient angular positioning of the forehead support with respect to the mask body so as to ensure efficient gas tightness (seal) and/or increase comfort for the user.

The solution of the present invention concerns a respiratory mask, in particular a nasal mask, comprising a mask body with an internal chamber, an expansion part being arranged on the mask body and projecting upwardly from said mask body, said expansion part comprising a traversing orifice, the expansion part and the mask body being molded in one-piece so that the expansion part is integral with the mask body, and a holding arm pivotable around a first axis located on said mask body or on said expansion part, characterized in that it further comprises a forehead support fixed to said holding arm and pivotable around an axis (BB) located on said holding arm, and an acting piece mobile in the traversing orifice of the expansion part, wherein the acting piece cooperates with the holding arm for pivoting said holding arm around the first axis (AA) when said acting piece moves in the traversing orifice of the expansion part, and a rotating knob cooperating with the acting piece for moving said acting piece in the traversing orifice when said rotating knob is operated by a user.

The mask according to the present invention can further comprise one or more of the following additional features:
- the holding arm is fixed to the expansion part and pivotable around a first axis AA located on said expansion part.
- the acting piece acts on the holding arm for changing its angular position around the first axis AA.
- the forehead support pivots around a second axis BB arranged on the holding arm.
- the rotating knob comprises a first threading cooperating with a second threading arranged on the acting piece.
- the holding arm pivots around the first axis AA of 50° or less.
- the forehead support pivots around the second axis BB of 90° or less.
- the expansion part is integral with the mask body, preferably the expansion part (2) and the mask body (1) are molded in one piece.
- the forehead support comprises one or several pillows of soft material.
- the mask body further comprises a gas inlet orifice in fluid communication with said internal chamber, said internal chamber further comprising a peripheral border and a cushion being fixed to said peripheral border, said cushion having a central aperture for receiving at least part of the patient's nose.
- the cushion arranged on the peripheral border of the internal chamber comprises one or several flexible membranes.
- the threading of the acting piece comprises a restriction.
- it further comprises a headgear and fixing means for fixing the headgear to the mask body.
- a mobile closing flap is connected to the shell and is able to move between at least a first position wherein said mobile closing flap is, at least partially, covering the lodging, and a second position wherein said mobile closing flap is not covering the lodging. The closing flap is mobile in rotation or is pivoting around a connecting element. The axis of the oxygen port is parallel to the axis of the gas inlet orifice of the shell. The oxygen port comprises a main hollow body with a central hollow passage linking an oxygen inlet situated in the lodging to an oxygen outlet situated in the breathing chamber. The lodging is situated in the upper part of the shell between the gas inlet orifice and a front support integral with the shell. The shell, the oxygen port and the lodging are molded in one piece. The closing flap is made of a unique piece of plastic, silicone or any other polymeric or elastomeric material. The lodging is arranged in the shell wall so as to project toward the internal chamber.

A preferred embodiment of a nasal mask according to the present invention is shown in the enclosed Figures, among which :
- Figure 1 represents a nasal mask according to the present invention,
- Figure 2 is a disassembled view of the mask of Figure 1,
- Figure 3 shows the angular courses of the holding arm and the forehead support,
- Figures 4a and 4b illustrate a forward motion of the acting piece of the mask of Figure 1,
- Figure 5 shows the rotative knob of the mask of Figure 1 and
- Figure 6 represents a enlarged with of the threading of the acting piece of the mask of Figure 1.

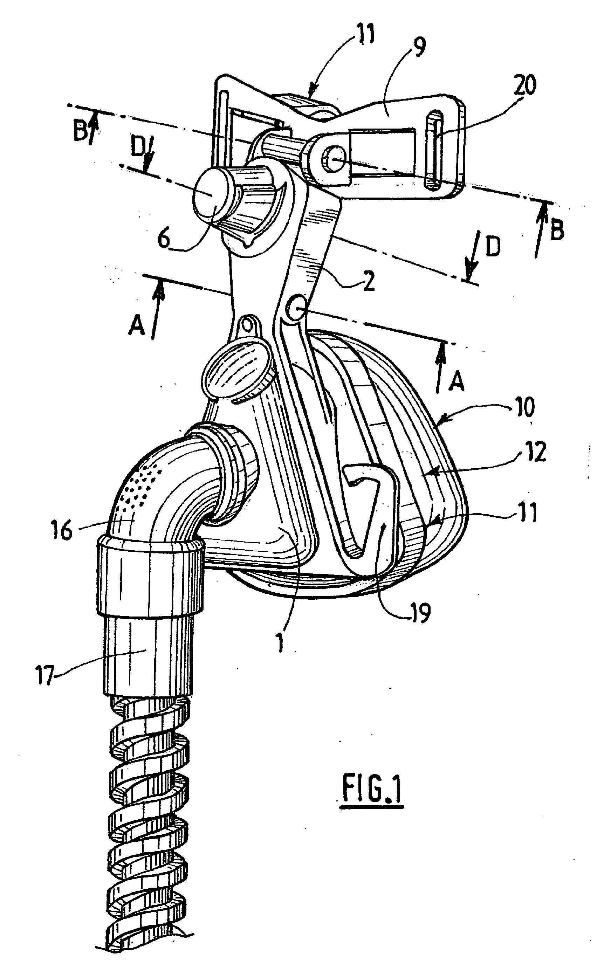

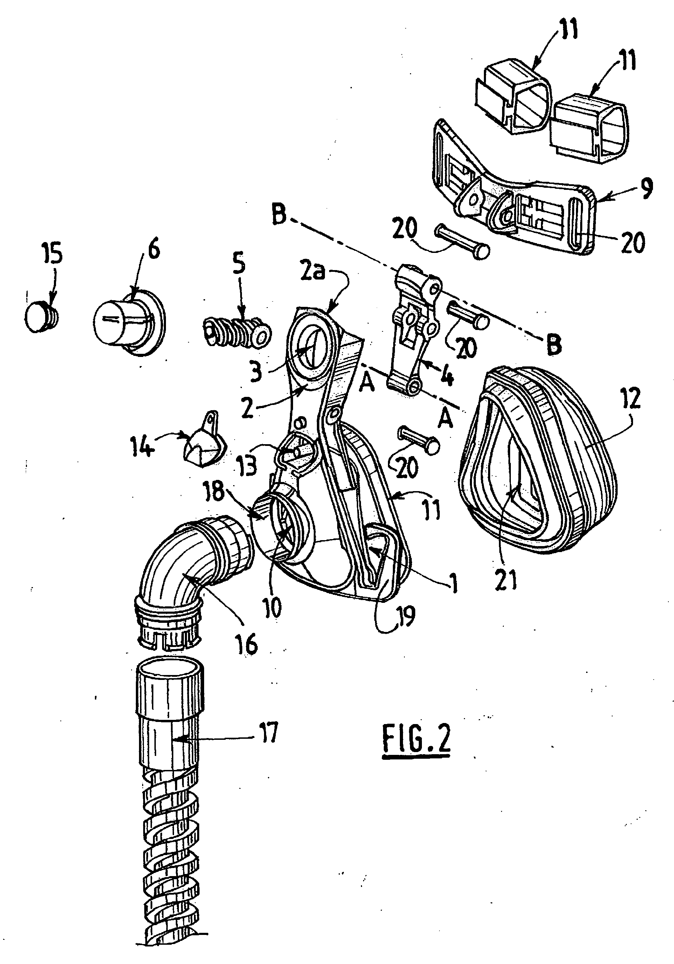

As illustrated in Figures 1 and 2, a respiratory mask according to the present invention comprises a rigid or semi-rigid hollow shell or mask body 1 defining an internal breathing chamber 10 or volume, wherein respiratory gas, such as air under pressure, is introduced via an inlet port 18 to which is connected a gas feeding line 17 by means of a tubular connector 16. The gas inlet orifice 18 is arranged at the center of the mask body 1 and through its wall thereby allowing air during pressure to be introduced in the breathing chamber 10.

The mask body 1 is preferably made of a polymer material, such as polycarbonate (PC), polypropylene (PP), ABS, nylon or polystyrene (PS), and is configured so as to be able to receive at least a part of the patient's nose. In other words, the patient introduces his/her nose into the internal volume of the breathing chamber 10 of the mask body 1 and breathes the pressurized gas contained therein.

The mask body 1 has preferably a general triangular tridimensional shape as visible in Figure 1 for example.

The mask body 1 further comprises an expansion part 2 arranged on the top of the mask body 1 and projecting upwardly from said mask body 1, i.e. projecting away from the external surface of the mask body 1. The expansion part 2 comprises a traversing orifice 3, preferably located at its distal end 2a. Preferably, the mask body 1 and the expansion 2 are made of polymeric material that is molded in one piece so as to obtain an expansion part 2 that is integral with the mask body 1.

Further, a forehead support 9 is connected to the mask body 1 by means of a pivotable holding arm 4, i.e. an arm 4 that is mobile in rotation, around a first axis AA situated either on the mask body 1 or on an expansion part 2, preferably on the expansion part 2. The forehead support 9 can comprise one or several pillows 11 of soft and comfortable material.

According to the present invention, an acting piece 5 is arranged in the traversing orifice 3 of the expansion part 2 and is mobile, preferably in translation, in said traversing orifice 3.

More precisely, the acting piece 5 cooperates with the holding arm 4 for pivoting said holding arm 4 around the first axis AA when said acting piece 5 moves in the traversing orifice 3 of the expansion part 2. Further, the forehead support 9 also pivots around a second axis BB arranged on the holding arm 4.

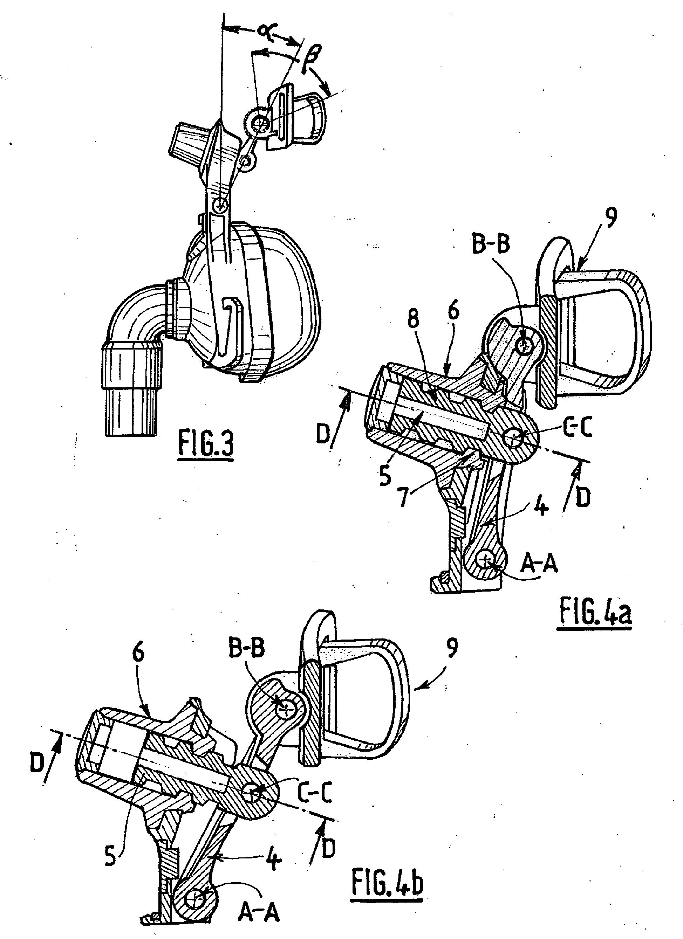

When a user acts on the acting piece 5 and moves it in the traversing orifice 3, said acting piece 5 acts on the holding arm 4 for changing its angular positioning of an angle a of preferably between 0 and 60°, as illustrated in Figure 3, thereby either approaching the support 9 to the forehead of the user, when the mask is carried by said user, or changing the pressure applied by the mask on the upper part of the nose of the user, when the forehead support 9 is already in contact with the forehead of the patient.

The angular orientation (angle β) of the forehead support 9 can be modified as it is freely pivoted around the second axis BB, thereby automatically matching the shape of the patient's forehead. For obtaining a correct orientation of the support 9 with respect to the patient's forehead, the support 9 should be able to pivot at an angle β of from between 0 and 90°.

The possibility of orienting the forehead support 9 and of changing the angular positioning of the holding arm 4 allows obtaining a correct orientation and positioning of the forehead support 2 carried by the holding arm 2, whatever is the morphology of the face of the user.

Actually, the back or forward motion of the acting piece 5 in the traversing orifice 3 is obtained by rotation of a rotating knob 6 cooperating with the acting piece 5, when said rotating knob 6 is manually operated by the user. For instance, in Figures 4a and 4b, the forward motion of the acting piece 5 has been illustrated.

As one can see in Figure 4a and 4b, the rotating knob 6 of axis DD comprises a first internal threading 7 that cooperates with a second external threading 8 arranged on the external surface of the acting piece 5.

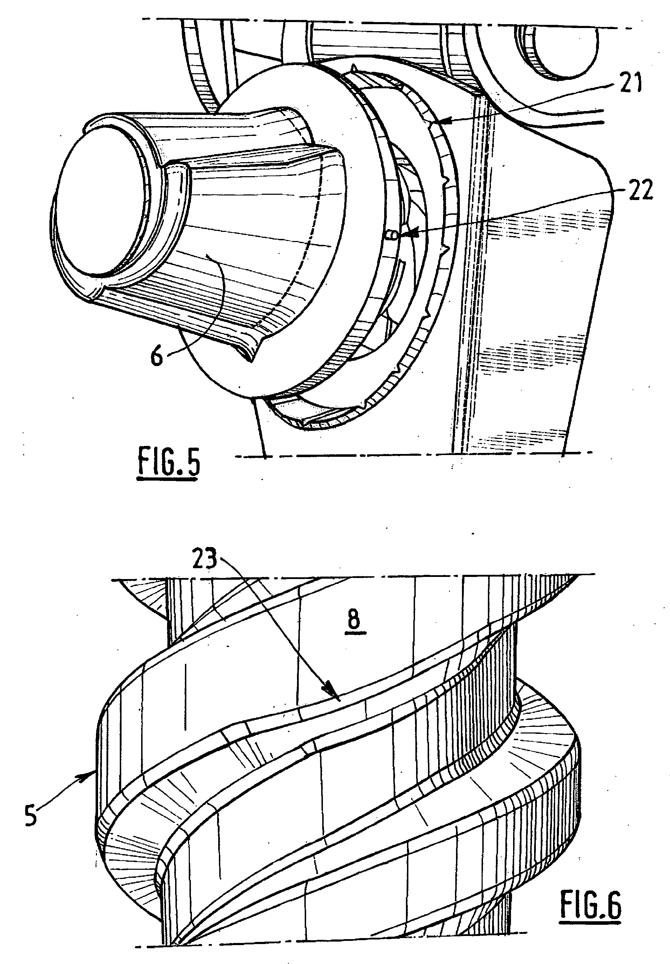

As visible in Figure 5, several notches 21 are arranged in the periphery of the traversing orifice 3 of the expansion part 2, whereas one (or several) pin 22 is arranged on the periphery of the rotating knob 6 and cooperates with the notches 21 for selecting the angular position of the holding arm 4. Indeed, each notch 21 corresponds to an angular position of the holding arm 4 and two successive notches 21 are spaced apart of a given distance corresponding to a given angle, for instance 10°. Hence, when the user turns the knob 6, the pin 22 is positioned in a given notch 21 corresponding to the desired angular position, and holds the knob 6 is said position.

The maximum course of the knob 6 is about 360° or less around its axis DD in the anticlockwise way.

As shown in Figure 6, the threading 8 of the acting piece 5 comprises a restriction 23 so as to block the rotation of the knob 6 by the user, when the start or the end of its course is close, i.e. at the beginning or at the end of the motion of rotation.

Furthermore, in order to ensure a tight positioning of the nasal mask on the patient's face and to increase the comfort for the patient, the peripheral border or edge 11 of the mask body 1 comprises a cushion 12 made of soft, resilient, elastomeric material that comes into contact with the patient's face. Said cushion 12 has a central aperture 21 for receiving at least a part of the patient's nose.

More precisely, the border 11 and the cushion 12 have a general triangular or saddle-shape structure so as to match the contours of the nasal region of the patient and hence the cushion 12 comprises, roughly speaking, an upper nasal bridge region, a lower region and cheek regions connecting the nasal bridge and lower regions. The cushion 4 can comprise one or several membranes, preferably two membranes. Such cushion 4 and mask body 1 structures are well-known in the art and taught by many documents, such as EP-A-1334742, EP-A-264772, EP-A-956069, EP-A-874667, US-A-2,931,356 or EP-A-1479406.

A headgear with straps (not shown) can be connected to the mask body 1, by fixing means 19, 20, such as fixing hooks 19 and fixing slots 20, for fixing the straps of the headgear and thereby maintaining the mask in the selected position on the head of the patient during its use and thus obtaining an efficient treatment of sleep disorders, such as OSA or similar disorders. The headgear-fixing hooks 19 are fixed and made integral with the shell 1 body. Preferably, the headgear-fixing hooks 19 are flat parallel portions, but of course, the headgear-fixing hooks 19 can have other forms. Such a mask structure is also well known in the art and disclosed in many documents such as, for example, EP-A-1334742, EP-A-462701, EP-A-1985327 or EP-A-956069.

The shell or mask body 1 is fluidly linked to a gas supply line 17, such as a flexible hose, by means of the tubular hollow connector 16, which delivers a respiratory gas, such as air under pressure, into the breathing chamber 10 of the shell 1. The tubular connector 16 comprises one or several venting outlets, such a several holes, for venting to the atmosphere the CO₂-containing gases exhaled by the patient. The gas supply line 17 is fed with air under pressure by a respiratory device or ventilator (not shown).

Furthermore, the mask body 1 comprises a lodging in recess arranged in the upper part of the mask 1 body, i.e., between the gas inlet orifice 18 and the expansion part 2, in which an oxygen and measurement port 13, i.e. an oxygen connection, is arranged. A closing flap 14 closes the lodging when the oxygen port 13 is not used as shown in Figure 1.

The nasal mask of the present invention can be used in a method for treatment of a respiratory disorder or condition, for example, in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask, in particular a nasal mask, comprising :
- a mask body (1) with an internal chamber (10),
- an expansion part (2) being arranged on the mask body (1) and projecting upwardly from said mask body (1), said expansion part (2) comprising a traversing orifice (3),
- the expansion part (2) and the mask body (1) being molded in one-piece so that the expansion part (2) is integral with the mask body (1), and
- a holding arm (4) pivotable around a first axis (AA) located on said mask body (1) or on said expansion part (2),
**characterized in that** it further comprises :
- a forehead support (9) fixed to said holding arm (4) and pivotable around an axis (BB) located on said holding arm (4),
- an acting piece (5) mobile in the traversing orifice (3) of the expansion part (2), wherein the acting piece (5) cooperates with the holding arm (4) for pivoting said holding arm (4) around the first axis (AA) when said acting piece (5) moves in the traversing orifice (3) of the expansion part (2), and
- a rotating knob (6) cooperating with the acting piece (5) for moving said acting piece (5) in the traversing orifice (3) when said rotating knob (6) is operated by a user.

2. Respiratory mask according to Claim 1, **characterized in that** the acting piece (5) is mobile in translation in the traversing orifice (3).

3. Respiratory mask according to Claim 1 or 2, **characterized in that** the holding arm (4) is fixed to the expansion part (2) and pivotable around a first axis (AA) located on said expansion part (2).

4. Respiratory mask according to any one of the preceding Claims, **characterized in that** the acting piece (5) acts on the holding arm (4) for changing its angular position around the first axis (AA).

5. Respiratory mask according to any one of the preceding Claims, **characterized in that** the forehead support (9) pivots around a second axis (BB) arranged on the holding arm (4).

6. Respiratory mask according to any one of the preceding Claims, **characterized in that** the rotating knob (6) comprises a first threading (7) cooperating with a second threading (8) arranged on the acting piece (5).

7. Respiratory mask according to any one of the preceding Claims, **characterized in that** the holding arm (4) pivots around the first axis (AA) of 50° or less.

8. Respiratory mask according to any one of the preceding Claims, **characterized in that** the forehead support (9) pivots around the second axis (BB) of 90° or less.

9. Respiratory mask according to any one of the preceding Claims, **characterized in that** the expansion part (2) and the mask body (1) are made of polymeric material.

10. Respiratory mask according to any one of the preceding Claims, **characterized in that** the forehead support (9) comprises one or several pillows (11) of soft material.

11. Respiratory mask according to any one of the preceding Claims, **characterized in that** the mask body (1) further comprises a gas inlet orifice (18) in fluid communication with said internal chamber (10), said internal chamber (10) further comprising a peripheral border (11) and a cushion (12) being fixed to said peripheral border (11), said cushion (12) having a central aperture for receiving at least part of the patient's nose, preferably said cushion (12) comprises one or several flexible membranes.

12. Respiratory mask according to any one of the preceding Claims, **characterized in that** the threading (8) of the acting piece (5) comprises a restriction (23).

13. Respiratory mask according to any one of the previous claims, **characterized in that** it further comprises a headgear and fixing means (19, 20) for fixing the headgear to the mask body (1).

14. Respiratory mask according to Claim 9, **characterized in that** the polymeric material is chosen among polycarbonate, polypropylene, ABS, nylon or polystyrene.

## Patentansprüche

1. Atemmaske, insbesondere eine Nasenmaske, die Folgendes aufweist:
- einen Maskenkörper (1) mit einem Innenraum (10),
- ein Erweiterungsteil (2), welches auf dem Maskenkörper (1) angeordnet ist und nach oben aus dem Maskenkörper (1) herausragt, wobei das Erweiterungsteil (2) eine durchgehende Öffnung (3) aufweist,
- das Erweiterungsteil (2) und der Maskenköper (1) derart aus einem Stück ausgeformt sind, dass das Erweiterungsteil (2) in den Maskenkörper (1) integriert ist, und
- einen Haltearm (4), der um eine erste Achse (AA) herum drehbar an dem Maskenkörper (1) oder an dem Erweiterungsteil (2) positioniert ist,
**dadurch gekennzeichnet, dass** sie ferner Folgendes aufweist:
- eine Stirnstütze (9), die an dem Haltearm (4) befestigt ist und um eine Achse (BB) herum drehbar an dem Haltearm (4) positioniert ist,
- ein Betätigungsteil (5), welches in der durchgehenden Öffnung (3) des Erweiterungsteils (2) beweglich ist, wobei das Betätigungsteil (5) mit dem Haltearm (4) zum Drehen von dem Haltearm (4) um die erste Achse (AA) herum zusammenwirkt, wenn sich das Betätigungsteil (5) in der durchgehenden Öffnung (3) von dem Erweiterungsteil (2) bewegt, und
- einen Drehknopf (6), der mit dem Betätigungsteil (5) zum Bewegen des Betätigungsteils (5) in der durchgehenden Öffnung (3) zusammenwirkt, wenn der Drehknopf (6) durch einen Benutzer bedient wird.

2. Atemmaske nach Anspruch 1, **dadurch gekennzeichnet, dass** das Betätigungsteil (5) in der durchgehenden Öffnung (3) in Translation beweglich ist.

3. Atemmaske nach Anspruch 1 oder 2, **dadurch gekennzeichnet, dass** der Haltearm (4) an dem Erweiterungsteil (2) befestigt ist und um eine erste Achse (AA) herum drehbar an dem Erweiterungsteil (2) positioniert ist.

4. Atemmaske nach einem der vorhergehenden Ansprüche, **dadurch gekennzeichnet, dass** das Betätigungsteil (5) an dem Haltearm (4) wirkt, um seine Winkelposition um die erste Achse (AA) herum zu verändern.

5. Atemmaske nach einem der vorhergehenden Ansprüche, **dadurch gekennzeichnet, dass** sich die Stirnstütze (9) um eine zweite Achse (BB) herum dreht, die an dem Haltearm (4) angeordnet ist.

6. Atemmaske nach einem der vorhergehenden Ansprüche, **dadurch gekennzeichnet, dass** der Drehknopf (6) ein erstes Gewinde (7) aufweist, welches mit einem zweiten Gewinde (8) zusammenwirkt, das an dem Betätigungsteil (5) angeordnet ist.

7. Atemmaske nach einem der vorhergehenden Ansprüche, **dadurch gekennzeichnet, dass** sich der Haltearm (4) um die erste Achse (AA) herum um 50° oder weniger dreht.

8. Atemmaske nach einem der vorhergehenden Ansprüche, **dadurch gekennzeichnet, dass** sich die Stirnstütze (9) um die erste Achse (BB) herum um 90° oder weniger dreht.

9. Atemmaske nach einem der vorhergehenden Ansprüche, **dadurch gekennzeichnet, dass** der Erweiterungsteil (2) und der Maskenkörper (1) aus einem Polymermaterial hergestellt sind.

10. Atemmaske nach einem der vorhergehenden Ansprüche, **dadurch gekennzeichnet, dass** die Stirnstütze (9) ein oder mehrere Kissen (11) aus weichem Material aufweist.

11. Atemmaske nach einem der vorhergehenden Ansprüche, **dadurch gekennzeichnet, dass** der Maskenkörper (1) ferner eine Gaseinlassöffnung (18) aufweist, die sich in Fluidverbindung mit dem Innenraum (10) befindet, wobei der Innenraum (10) ferner eine Umfangseinfassung (11) und ein Kissen (12) aufweist, das an der Umfangseinfassung (11) befestigt ist, wobei das Kissen (12) eine zentrale Aussparung zur Aufnahme von mindestens einem Teil der Nase des Patienten aufweist, wobei das Kissen (12) vorzugsweise eine oder mehrere flexible Membranen aufweist.

12. Atemmaske nach einem der vorhergehenden Ansprüche, **dadurch gekennzeichnet, dass** das Gewinde (8) des Betätigungsteils (5) eine Einengung (23) aufweist.

13. Atemmaske nach einem der vorhergehenden Ansprüche, **dadurch gekennzeichnet, dass** sie ferner ein Kopfband und Befestigungsmittel (19, 20) zum Befestigen des Kopfbandes an dem Maskenkörper (1) aufweist.

14. Atemmaske nach Anspruch 9, **dadurch gekennzeichnet, dass** das Polymermaterial ausgewählt ist aus Polycarbonat, Polypropylen, ABS, Nylon oder Polystyrol.

## Revendications

1. Masque respiratoire, en particulier un masque nasal, comprenant :
- un corps de masque (1) avec une chambre interne (10) ;
- une partie d'extension (2) étant agencée sur le corps de masque (1) et se projetant vers le haut depuis ledit corps de masque (1), ladite partie d'extension (2) comprenant un orifice traversant (3),
- la partie d'extension (2) et le corps de masque (1) étant moulés en une pièce de manière à ce que la partie d'extension (2) fasse partie intégrale du corps de masque (1), et
- un bras porteur (4) pouvant pivoter autour d'un premier axe (AA) situé sur ledit corps de masque (1) ou sur ladite partie d'extension (2),
**caractérisé en ce qu'**il comprend, en outre :
- un support frontal (9) fixé audit bras porteur (4) et pouvant pivoter autour d'un axe (BB) situé sur ledit bras porteur(4),
- une pièce d'actionnement (5) mobile dans l'orifice traversant (3) de la partie d'extension (2), où la pièce d'actionnement (5) coopère avec le bras porteur (4) pour faire pivoter ledit bras porteur (4) autour du premier axe (AA) quand ladite pièce d'actionnement (5) se déplace dans l'orifice traversant (3) de la partie d'extension (2), et
- un bouton rotatif (6) coopérant avec la pièce d'actionnement (5) pour déplacer ladite pièce d'actionnement (5) dans l'orifice traversant (3) quand un utilisateur actionne ledit bouton rotatif (6).

2. Masque respiratoire selon la revendication 1, **caractérisé en ce que** la pièce d'actionnement (5) est mobile en translation dans l'orifice traversant (3).

3. Masque respiratoire selon la revendication 1 ou 2, **caractérisé en ce que** le bras porteur (4) est fixé à la partie d'extension (2) et peut pivoter autour d'un premier axe (AA) situé sur ladite partie d'extension (2).

4. Masque respiratoire selon l'une quelconque des revendications précédentes, **caractérisé en ce que** la pièce d'actionnement (5) agit sur le bras porteur (4) pour modifier sa position angulaire autour du premier axe (AA).

5. Masque respiratoire selon l'une quelconque des revendications précédentes, **caractérisé en ce que** le support frontal (9) pivote autour d'un second axe (BB) agencé sur le bras porteur (4).

6. Masque respiratoire selon l'une quelconque des revendications précédentes, **caractérisé en ce que** le bouton rotatif (6) comprend un premier filetage (7) coopérant avec un second filetage (8) disposé sur la pièce d'actionnement (5).

7. Masque respiratoire selon l'une quelconque des revendications précédentes, **caractérisé en ce que** le bras porteur (4) pivote autour du premier axe (AA) de 50° ou moins.

8. Masque respiratoire selon l'une quelconque des revendications précédentes, **caractérisé en ce que** le support frontal (9) pivote autour du second axe (BB) de 90° ou moins.

9. Masque respiratoire selon l'une quelconque des revendications précédentes, **caractérisé en ce que** la partie d'extension (2) et le corps de masque (1) sont formés d'un matériau polymère.

10. Masque respiratoire selon l'une quelconque des revendications précédentes, **caractérisé en ce que** le support frontal (9) comprend un ou plusieurs coussinet(s) (11) en matériau mou.

11. Masque respiratoire selon l'une quelconque des revendications précédentes, **caractérisé en ce que** le corps de masque (1) comprend, en outre, un orifice d'arrivée de gaz (18) en communication fluidique avec ladite chambre interne (10), ladite chambre interne (10) comprenant, en outre, une bordure périphérique (11) et un coussin (12) fixé à ladite bordure périphérique (11), ledit coussin (12) ayant une ouverture centrale pour recevoir au moins une partie du nez du patient, de préférence ledit coussin (12) comprenant une ou plusieurs membranes souples.

12. Masque respiratoire selon l'une quelconque des revendications précédentes, **caractérisé en ce que** le filetage (8) de la pièce d'actionnement (5) comprend une restriction (23).

13. Masque respiratoire selon l'une quelconque des revendications précédentes, **caractérisé en ce qu'**il comprend, en outre, un harnais de tête et un moyen de fixation (19, 20) servant à fixer le harnais de tête au corps de masque (1).

14. Masque respiratoire selon la revendication 9, **caractérisé en ce que** le matériau polymère est choisi parmi le polycarbonate, le polypropylène, l'ABS, le nylon ou le polystyrène.
